# EUROPEAN PATENT APPLICATION

(11) **EP 2 179 733 A1**
(43) Date of publication of application: **28.04.2010**
(21) Application number: 08793783.5
(22) Date of filing: 25.07.2008
(51) Int. Cl.: A61K 31/496, A61K 31/265

(54) **PHARMACEUTICAL COMPOSITION COMBINING AN ANTIMICROBIAL AGENT AND A SELECTIVE ENKEPHALINASE ENZYME INHIBITOR, WHICH CAN BE USED TO CONTROL AND TREAT ACUTE BACTERIAL DIARRHOEA**

(30) Priority: 27.07.2007 MX 2007009121
(71) Applicant: World Trade Import-Export, WTIE A.G., 6302 Zug (CH)
(72) Inventor: GARCÍA ARMENTA, María Elena, Jalisco (MX); SANTOS MURILLO, Josefina, Jalisco (MX); ALVAREZ OCHOA, Víctor Guillermo, Jalisco (MX)
(74) Representative: Carvajal y Urquijo, Isabel
(86) International application number: PCT/MX2008/000100
(87) International publication number: WO 2009/022893

(57) **Abstract**

The invention relates to a pharmaceutical composition comprising the synergic combination of an antimicrobial agent such as ciprofloxacin and a selective enkephalinase enzyme inhibitor such as racecadotril, as well as to pharmaceutically acceptable excipients, which are formulated in a single dosage unit to be administered orally. The invention is intended to control and treat acute bacterial diarrhea, providing an improved therapeutic effect, faster onset of action and reduced risk of serious complications.

## Description

### FIELD OF INVENTION

The present invention is applicable in pharmaceutical industry and describes a pharmaceutical composition comprising a synergistic combination of an antimicrobial agent, such as active principle: Ciprofloxacin and an enkephalinase enzyme selective inhibitor agent, such as active principle: Racecadotril, in addition to pharmaceutically acceptable excipients; which are formulated in a single dosage unit to be orally administered, which is indicated for bacterial origin acute diarrhea control and treatment.

The combination of above mentioned active principles produces a higher synergistic effect when administered in combination in a single dosage unit unlike when these are independently administered, causing benefits such as: lower active principle concentrations comprised in the formula, lower administered doses, faster action, therapeutic effect maximization and less risks for side effects.

### BACKGROUND OF INVENTION

More than 90% of diarrhea cases are caused by the presence of infective agents and the remaining 10% is due to drug consumption, toxic substance intake, ischemia and other processes.

*Diarrhea* is defined as an increase in volume (increase in the amount of evacuated feces), fluidity (consistency of fecal matter becomes aqueous) and frequency in intestinal motility. When these symptoms are sudden and together with stomach pain or colic, the individual is said to manifest an acute diarrhea.

Acute diarrhea (DA) is defined as a condition characterized by the presence of increased stools in frequency (more than 2 to 3 along a day), with consistency alteration (generally soft or liquid), whether or not associated with general symptoms (fever, shiver, nausea or abdominal colic) and during no more than one week.

Bacterial acute diarrhea is predominantly acquired by fecal or oral route when drinking water and/or eating contaminated food by a number of bacterial microorganisms.

Most of acute diarrhea episodes are self-limited, and though more than 90% are produced by infective agents, in most cases the specific microorganism responsible of infection is not easily identified in fecal matter.

Diarrhea-causing microorganisms exert their action through: a) enterotoxins which act over enterocyte secretory function, causing a coliform syndrome and b) invasion of digestive mucosa causing ulcerations and acute inflammatory reaction, which causes a dysenteriform syndrome.

Food poisoning is caused by toxin intake synthesized by the bacterial microorganism present in food, produced previously to its consumption, for example, those produced by thermostable enterotoxins such as: *Staphilococcus aureus, Clostridium perfringens* and *Bacillus cereus.* In these situations, the microbiological diagnosis is performed by bacteria culture in suspected food. Main manifested symptoms in an acute diarrhea condition are:
- Profuse evacuations with dehydration
- Soft or aqueous fecal matter with presence of microscopic blood.
- Fever above 38.5°C, nausea and vomit.
- Intense abdominal pain and colic. Persistency of clinical condition along 48 hours without improvement.
- New infection outbreaks manifested in the community.
- Susceptibility in immunosuppressed individuals or in those older than 50 years.
It is important to distinguish from the beginning between inflammatory and non-inflammatory diarrhea.

*Non-inflammatory diarrhea* is usually characterized by liquid evacuations in large volume (more than 1 liter per day), but without any blood or pus. Even when patient usually suffers stomach cramps, intense and sustained abdominal pain is not usual and fever is not commonly manifested. This diarrhea is usually produced by bacteria such as: enterotoxic *E. coli, Vibrio cholerae,* staphylococcus or *Clostridium.*

*Inflammatory diarrhea* is characterized by very frequent evacuations with little volume, with the presence of mucus or blood and which may be accompanied by tenesmus, fever or severe abdominal pain. When this diarrhea is of infectious origin there is an increased presence of leukocytes in fecal matter and is usually caused by the presence of bacterial microorganisms such as: *Salmonella, Shigella,* enterohemorrhagic *E. coli, Clostridium difficile, Entamoeba histolytica* or *Yersinia.*

Acute bacterial fever dysentery is more frequently caused by *Shigella, Salmonella,* enteroinvasive *Escherichia coli, Campylobacter, Yersinia* or *Clostridium.* Dysentery is characterized by the presence of: faeces with mucus, pus, rectal bleeding, abdominal pain and cramps, fever, cephalea and general discomfort. There is usually little dehydration except in those cases with *Shigella dysenteriae* infection. Bacteria are produced in small intestine and invade large intestine.

There is a water and electrolyte absorption in small intestine through epithelial villus and simultaneously a secretion thereof through crypts.

Absorption is normally higher than secretion, therefore more than 90% of fluids which reach small intestine are absorbed along the gastrointestinal tract; if a change is produced in bidirectional flow, that is, absorption is decreased or secretion is increased, the volume which reaches large intestine may overcome the absorption capacity thereof, this being the mechanism whereby diarrhea is produced.

Acute diarrhea may be basically produced by two mechanisms which may be superimposed in a single individual, such as: Secretion = secretory diarrhea and osmotic action = osmotic diarrhea. Osmotic diarrhea is caused by the presence of non-absorbable solutes in intestinal lumen, such as laxatives and misdigested food which cause water output. It disappears with fasting. It is frequent after oral contrast medium delivery for TAC performance. Secretory diarrhea is secondary to active ion secretion which causes a remarkable water loss. Diarrheas produced by virus (rotavirus), bacterial enterotoxins (cholera, E. coli), protozoos (giardia), AIDS-associated disorders, intestinal vasoactive peptide (VIP) producing tumors, carcinoid tumors (histamine and serotonin) and distal colon hairy adenomas are within this group. It does not disappear with fasting.

Escherichia coli enterotoxic strains produce toxins which induce a high water and electrolyte intestinal secretion. In this regard, referred mechanism is qualitatively similar to that produced by *Vibrio cholerae;* however, the latter causes even higher fecal losses (by secretory mechanism exacerbation).

*Escherichia coli* enteroadherent strains by closely adhering to intestinal mucosa may destroy brush border over surface cells where implanted. Invasive cells of *Escherichia coli* and *Shigella* invade mucosa, the latter being in charge of preparing a secretogenic toxin. *Clostridium difficile,* which is usually associated with the use of antibiotics also produces toxins, those which affect large intestine.

Acidosis dehydration is the most common consequence of acute diarrhea. In predominantly secretory diarrhea, intestinal content shows high sodium and chloride concentrations. In predominantly osmotic diarrheas, electrolyte content is lower. In these cases, fecal content osmolarity mainly depends on the presence of organic substrates (unabsorbed carbohydrates, middle chain organic acids, etc.).

Most of diarrheas which cause excessive liquid losses result in an isotonic concentration in body spaces (isonatremia).

A "mild or non-apparent" dehydration (which is the most frequently produced in acute diarrheas), is reported when the loss of body water is lower than 40 to 50 mL/kg body weight. Up to about this limit, objective clinical signs of dehydration are a few. Firstly, dryness is not yet observed in mucosa or a decrease in tears; however, there may be an increase in pulse rate, certain skin paleness and patient is seen someway thirsty. In these cases it is correct to talk about "non-apparent" dehydration because in spite of a loss of body water, this has not been present in the objective indexes of physical examination.

When dehydration reaches a body liquid loss from 50 to 100 mL./kg of body weight, it is commonly referred to as "mild" dehydration. When losses are above 100 mL/kg of body weight it is referred to as "severe" dehydration. Acidosis is relatively proportional to dehydration rate.

Acute diarrheic infections are currently a frequent cause of medical visit for primary attention. Diagnosis is generally clinic, condition characteristics are oriented to causal agent identification and the background to acknowledge how infection was acquired provide the way to issue an accurate diagnosis. In disease clinical history is quite important to investigate: duration of diarrhea, stool characteristics; stool frequency along the prior 24 hours; vomit presence and frequency, fever presence, irritability, malaise, thirst; ability for accepting or not food and liquids; type and volume of received food; normality or not of diuresis, etc. The lack of knowledge of said information causes that in many cases an antibiotic and antidiarrheal treatment shall be empirically started.

Treatment shall take into account support measures such as hydration with an equilibrated supply of glucose and electrolytes. In most cases, treatment includes antidiarrheals administration and in any event, antibiotics, especially in acute diarrheas together with fever, dehydration, general status compromising or dysenteric syndrome, in patients older than 60 years.

Most of medicaments which are currently found in marketplace for treatment of bacterial acute diarrheas comprise active principles which are independently formulated, which fulfill with a so specific therapeutic activity that in most cases is limited as to the microorganism diversity which are present and which cause an infectious acute diarrhea.

### SUMMARY OF INVENTION

In order to provide a pharmaceutical alternative which achieves to remove the presence of pathogen agents found in infectious acute diarrheas and which further may arrest the bacterial growth, thus achieving a control of evacuation frequency and amount which leads to liquid and electrolyte loss compromising patient integrity, the development of present invention was carried out, wherein a pharmaceutical composition comprising a combination of an antimicrobial agent and an enkephalinase selective inhibitor agent is described, those which act synergistically and which are formulated in a single dosage unit to be orally administered, providing benefits such as: lower active principle concentrations comprised in the formula, lower administered doses, therapeutic effect maximization, faster action, reduction of a possibility to show complications and risk reduction in side effect manifestation.

### DETAILED DESCRIPTION OF INVENTION

Antimicrobials are defined as natural (fungi or bacteria), synthetic or semi-synthetic origin chemical substances having the capability of inhibiting pathogen microorganism development or to cause a destruction thereof, those which are capable of producing an infection. They are systemic use agents which reduce and control the presence of contaminant microorganisms in host.

It is fundamental for an antibiotic to exert its action, to reach the infected site, penetrating in bacteria (by diffusion or active transport) and intracellularly reaching the required concentration.

Once within cells, the antibiotic may exert two types of effect: bacteriostatic, which prevents microorganism development without causing its destruction, being able to multiply again upon effect disappearance; or bactericide, wherein microorganism destruction is produced (lethal effect). Antibiotics exert their action through the following specific mechanisms or functions: a) inhibition of cell wall synthesis; b) alteration over cytoplasm membrane or permeability; c) Inhibition of protein synthesis and d) Blockage or inhibition of nucleic acid synthesis.

Quinolones are a known family of antibiotics since 60's. First used quinolone was nalidixic acid which, together with pipemidic acid, comprises the first generation of quinolones.

Second generation quinolones are fluorinated derivatives or fluoroquinolones (FQ). There is a third generation comprised by bi- and trifluorinated derivatives and those of four generation are currently under development.

First FQ in appearing was Norfloxacin, which represented a significant pharmaceutical advance due to its higher power and antibacterial spectrum. The following came afterwards: Ciprofloxacin, Ofloxacin, Enoxacin, Lomefloxacin, Temafloxacin, Levofloxacin, Sparfloxacin, Trovafloxacin, Grepafloxacin, Gatifloxacin, Moxifloxacin and Gemifloxacin. Some of them were withdrawn from market after their marketing was approved or their use has been restricted due to toxic effects (Sparfloxacin, Trovafloxacin, Grepafloxacin).

First quinolones had activity only against gram-negative aerobic bacteria and they were efficient for treatment of gastrointestinal and urinary infections. The introduction of a fluorine atom into quinolone basic molecule lead to fluoroquinolones (FQ), having a more powerful activity, higher antibacterial spectrum, longer half-life and excluding Norfloxacin, they achieve good serum levels whereby treatment of systemic infections is possible.

FQ are bacterial agents which act by inhibiting ADN-girase, an enzyme which participates in double-stranded DNA helix folding and being fundamental for genetic material three-dimensional structure, exerting their action at intracellular level.

Their activity basically depends on two factors such as: the capability of traversing cytoplasm barrier and the affinity by DNA-girases from bacteria, which allows achieving the same or higher tissue concentrations to those achieved in serum; leading to higher concentrations at intestinal level.

Ciprofloxacin has a broad "in vitro" activity spectrum and good activity before (-)gram bacteria.

Orally administered ciprofloxacin absorption is 95% in two hours and 100% in three hours; providing from 70% to 80% bioavailability and maximum blood concentrations are approximately achieved one hour after delivery. Ciprofloxacin provides a high distribution volume and reaches concentrations quite higher than in serum in a number of tissues and liquids. Its dosage-independent half-life is 4 hours and is bound to plasma proteins in 30%.

Ciprofloxacin is mainly cleared by renal route, through glomerular filtering and tubular excretion as Ciprofloxacin without any change and in the form of its four active metabolites: oxiciprofloxacin, sulfociprofloxacin, desmetilciprofloxacin and formilciprofloxacin. Hepatobilliary system is an alternative clearance route.

Although oral rehydration therapy constitutes a basic aspect for acute diarrhea treatment, a number of drugs have been currently assessed which provide interesting benefits and which function as a supplementary support in patient's recovery who suffer said infection.

Activity of enkephalinase inhibiting agents is translated at intestinal mucosa level in an effect maintained by enkephalins over delta-type (δ) opioid receptors, involved in water and electrolyte intestinal reuptake regulation. Action maintained by enkephalins over such receptors is responsible of water and electrolyte intestinal antisecretory effect, neutralizing the hypersecretion induced by several chemical and biologic agents.

Peripheral enkephalinase inhibition leads to an increase in enkephalins permanence, with the consequent regulating effect over intestinal secretion.

The first step for treatment of acute diarrhea was opioid drug introduction, such as morphine, codeine and loperamide, as possible pharmacological options. However, such drugs may produce an intestinal slowness mediated by µ receptors, constipation or effects in central system such as sleepiness and respiratory depression, because of their capacity to traverse hematoenkephalic barrier. Therefore, the use of loperamide is restricted in certain conditions, such as: patients in childhood, dysentery or bowel inflammatory disease.

By mid-80's, Edelman theorized about the need of an antidiarrheal with antisecretory action which rapidly acts locally in intestinal mucosa without causing constipation (Edelman, 1985).

Because of that, at the beginning of 90's, trials started with Acetorphan drug, an intestinal enkephalinase enzyme inhibitor, a membrane metallopeptidase which is abundantly found on gastrointestinal tract villus, which main function is enkephalin degradation (endogenous opioids) (Baumer et al, 1992; Hinterleitner et al, 1997) .

Said drug was later named as Racecadotril, which active metabolite is known as Tiorphan, being the first physiological antidiarrheal representing a new pharmacotherapeutical group.

Racecadotril is an enkephalinase enzyme selective inhibitor belonging to a new class of drugs named "enkephalinase inhibitors". It is a diesterified lipophylic prodrug which is rapidly hydrolysed to become into its active metabolite named Tiorphan. It is indicated for supplementary symptomatic treatment of acute diarrheas and when oral rehydration and usual support measures are insufficient for controlling the clinical condition

Enkephalinases are membrane metallopeptidases which are located in gastrointestinal tract, in central nervous system and in other tissues, and they are responsible of enkephalin degradation (endogenous opioids).

Racecadotril action mechanism is based on enkephalinase inhibition in gastrointestinal tract which is translated into an enkephalin antisecretory effect extension, reducing water and electrolyte hypersecretion towards intestinal lumen; thus achieving a physiological effect over water and electrolyte intestinal hypersecretion which causes dehydration, therefore being capable of reducing duration of diarrheic conditions, decreasing the number and volume of evacuations (Matheson and Noble, 1992) .

Besides the efficacy derived from its pharmacological properties, Racecadotril also shows a good safety profile which does not cause effects over central nervous system and neither has effects over intestinal motility.

Said antisecretory mechanism is different from that exerted by Loperamide, which is based on its decrease in intestinal transit speed decrease; on the other hand, Racecadotril does not modify the intestinal transit time, nor significantly affects baseline secretion.

Its action is exclusively peripheral (only distributed in tissues in 1% Racecadotril doses), without manifesting significant effects over central nervous system. When Racecadotril is orally administered, hematoencephalic barrier is not traversed but instead upon intravenous delivery activity induction over central nervous system (CNS) has been observed.

Duration and extension of Racecadotril effect are a function of administered doses. After oral administration, Racecadotril is rapidly absorbed, plasmatic enkephalinase inhibiting effect starts to be manifested 30 minutes after administration, reaching a maximum concentration at 60 minutes from administration and its maximum activity is manifested after 2 hours. With 1.5 mg/kg oral doses, maximum enzymatic inhibition level is about 90% and inhibiting effect over plasmatic enkephalinase is significantly maintained in about 8 hours.

It should be mentioned that Racecadotril bioavailability is not modified when administered with food, but maximum activity is delayed about one and half hours.

Only 1% of administered dose is distributed in tissues. 90% of Racecadotril active metabolite (Tiorphan) is attached to plasmatic proteins, mainly albumin.

Tiorphan active metabolite, which in turn is transformed into an inactive metabolite, is removed by renal, fecal and pulmonar route, being Racecadotril mean life about 3 hours.

There are a number of pharmaceuticals in marketplace which are directed to bacterial acute diarrhea treatment, wherein several active principles are found which are independently formulated; in these cases, it is quite important to prevent complications which are present with diarrhea and caused by liquid and electrolyte loss, which imminently are added to the already suffered infectious process; therefore, administration of a treatment comprising a combination of drugs having specific therapeutic activity and acting through several ways is indispensable, in order to shorten and/or eradicate both the infectious process and the liquid and electrolyte loss.

The pharmaceutical composition subject of present invention comprises a synergistic combination of an antimicrobial agent, such as active principle: Ciprofloxacin and an enkephalinase enzyme selective inhibitor agent, such as active principle: Racecadotril, in addition to pharmaceutically acceptable excipients, which are formulated in a single dosage unit to be orally administered in capsule or tablet form, which is indicated for treatment and control of bacterial acute diarrhea and which has been developed taking into account that both active principles have a great efficacy and capacity for attacking and removing the presence of several pathogen agents found in bacterial acute diarrhea in addition to arresting bacterial growth progression and reducing liquid and electrolyte loss in less time; thus preventing the appearance of severe complications which may cause death.

Antimicrobial agent used in the pharmaceutical composition subject of present invention such as active principle: Ciprofloxacin, is present in the formulation in a concentration range from 250.0 mg. to 1.0 g per dose unit.

The enkephalinase enzyme selective inhibitor agent used in the pharmaceutical composition subject of present invention such as active principle: Racecadotril, is present in the formulation in a concentration range from 100.0 mg to 300.0 mg per dose unit.

In order to assess the efficacy and tolerance of the pharmaceutical composition subject of present invention, as well as the synergistic effect of Ciprofloxacin and Racecadotril active principles combined in a single dosage unit, a comparative clinical study was carried out wherein above mentioned active principles were separately administered, as well as a combination thereof.

### COMPARATIVE CLINICAL STUDY BETWEEN CIPROFLOXACIN AND A COMBINATION OF CIPROFLOXACIN / RACECADOTRIL IN PATIENTS WITH ACUTE BACTERIAL DIARRHEA.

A double-blind, prospective, randomized comparative clinical study was carried out to compare Ciprofloxacin efficacy vs Racecadotril / Ciprofloxacin combination in patients with acute bacterial diarrhea with the primary objective of measuring diarrhea and bacterial condition duration.

50 patients who fulfilled with inclusion criteria were included in the study, which were:
- Age between 18 and 75 years
- Male and female
- Without concomitant diseases (diabetes mellitus, arterial hypertension).
- With bacterial origin acute diarrhea.
- Fever ≥ 38.5 °C, general discomfort, pain and/or abdominal distress, nausea and anorexia.

Patients were divided in two study groups for receiving:
Group 1: received Ciprofloxacin 500 mg every 12 hours.
Group 2: received a combination of Racecadotril 100 mg / Ciprofloxacin 500 mg every 12 hours.

Treatment was administered along 3 follow-up days.

Clinical histories were performed to patients, as well as physical examination to collect baseline data.

### RESULTS.

50 patients included in the study completed it; no side effects were present during study.

Baseline data did not show significant differences among groups.

**Table 1. Baseline data between groups.**

| | Group 1 | Group 2 |
|---|---|---|
| | (n=25) | (n=25) |
| Age | 29 ± 13.5 | 33 ± 11.2 |
| Sex (F/M) | 12/13 | 14/11 |
| Fever | 100% | 100% |
| General Discomfort | 100% | 100% |
| Abdominal Distress | 100% | 100% |
| Abdominal Pain | 100% | 100% |
| Nausea | 100% | 100% |
| Anorexia | 100% | 100% |
| Number of evacuations per day | ≥ 8 | ≥ 8 |

Results made evident that efficacy and promptness of drug administered in Group 2 was higher than in patients from Group 1.

**Table 2. Efficacy and promptness compared between Groups 1 and 2 at the end of study.**

| | Group 1 | Group 2 |
|---|---|---|
| Physical assessment (excellent or good in %) | 89.3 ± 0.3 | 98.0 ± 0.7* |
| Delay in diarrhea resolution (days) | 3.1 ± 0.2 | 1.9 ± 0.2* |
| Recovery after 72 hours (%) | 80.0 | 93.0* |
| Abdominal distress duration (days) | 2.0 | 1.0* |
| Abdominal pain > one day (%) | 55.3 | 37.2* |
| Later constipation (%) | 15.7 | 7.9* |
| Fever > one day (%) | 20.2 | 19.5 |
| Nausea (%) | 0 | 0 |
| Anorexia (%) | 0 | 0 |
| Number of evacuations per day | 3 | 1* |

| | | |
|---|---|---|
| * Significant differences | | |

### CONCLUSIONS.

Results demonstrate that a Ciprofloxacin / Racecadotril combination, enhances promptly and effectively patient conditions with bacterial origin acute diarrhea, compared with Ciprofloxacin administration alone.

This efficacy and promptness is caused by the synergistic activity of a combination at different levels, using lower active principle concentrations formulated in a single dosage unit; with lower risks than severe complications are present caused by an excessive loss of liquids and electrolytes.

## Claims

1. A pharmaceutical composition **characterized in that** comprises a synergistic combination of an antimicrobial agent, such as active principle: Ciprofloxacin and an enkephalinase enzyme selective inhibitor agent, this being the active principle: Racecadotril, in addition to pharmaceutically acceptable excipients; wherein active principles are found present in formulation in a concentration range from 250.0 mg to 1.0 g for Ciprofloxacin and from 100.0 mg to 300.0 mg for Racecadotril; those which are formulated in a single dosage unit to be orally administered, which is indicated for control and treatment of bacterial acute diarrhea.

2. A pharmaceutical composition according to claim 1, **characterized in that** antimicrobial agent such as active principle: Ciprofloxacin, is present in the formulation in a concentration range from 250.0 mg to 1.0 g, being preferably used in the formulation a concentration of 500.0 mg per dose unit.

3. A pharmaceutical composition according to claims 1 and 2, **characterized in that** enkephalinase enzyme selective inhibitor agent, such as active principle: Racecadotril, is present in the formulation in a concentration range from 100.0 mg to 300.0 mg, being preferably used in the formulation a concentration of 100.0 mg per dose unit.

4. A pharmaceutical composition according to claims 1 to 3, **characterized in that** is formulated in a single dosage unit to be orally administered in capsule or tablet form.

5. The use of a pharmaceutical composition according to claims 1 to 4, **characterized in that** is indicated for control and treatment of bacterial acute diarrhea.

6. A pharmaceutical composition according to claims 1 to 5, **characterized in that** manifests a significant efficacy and capacity for attacking and removing the presence of a number of pathogen agents found in bacterial acute diarrhea, in addition to arresting bacterial growth progression and reducing liquid and electrolyte loss in less time, thus preventing the appearance of severe complications with irreversible consequences.
